# EUROPÄISCHE PATENTSCHRIFT

(11) **EP 0 732 912 B1**
(45) Veröffentlichungstag und Bekanntmachung des Hinweises auf die Patenterteilung: **17.01.2001**
(21) Anmeldenummer: 95903306.9
(22) Anmeldetag: 30.11.1994
(51) Int. Cl.: A61K 7/48, A61K 47/10, A61K 47/14

(54) **KOSMETISCHE UND/ODER PHARMAZEUTISCHE ZUBEREITUNGEN MIT VERBESSERTEM HAUTGEFÜHL**
COSMETIC AND/OR PHARMACEUTICAL PREPARATIONS WITH IMPROVED SKIN FEEL
PREPARATIONS COSMETIQUES ET/OU PHARMACEUTIQUES A SENSATION CUTANEE AMELIOREE

(30) Priorität: 08.12.1993 DE 4341794
(43) Veröffentlichungstag der Anmeldung: 25.09.1996
(73) Patentinhaber: Cognis Deutschland GmbH, 40589 Düsseldorf (DE)
(72) Erfinder: ANSMANN, Achim, D-40699 Erkrath (DE); KAWA, Rolf, D-40789 Monheim (DE); MOHR, Klaus-Michael, D-42327 Wuppertal (DE); KOESTER, Josef, D-40221 Düsseldorf (DE)
(86) Internationale Anmeldenummer: EP9403974
(87) Internationale Veröffentlichungsnummer: WO9515743

(56) Entgegenhaltungen:
- EP-A- 0 453 603
- EP-A- 0 617 952
- WO-A-92/07543
- DE-U- 9 317 968
- GB-A- 780 801
- SEIFEN-ÖLE-FETTE-WACHSE, Bd. 117,Nr. 10, 1991 AUGSBURG, DE, Seiten 369-371, ANSMANN ET AL. 'COSMETIC WATER IN OIL EMULSIONS- HOW TO FORMULATE ELEGANT SKIN CARE PRODUCTS'

## Beschreibung

Die Erfindung betrifft neue kosmetische und/oder pharmazeutische Zubereitungen, die als Ölkörper Guerbetalkohole auf Basis von kurzkettigen Fettalkoholgemischen bzw. deren Ester mit ausgewählten Fettsäuren enthalten.

### Stand der Technik

Zur Herstellung kosmetischer bzw. pharmazeutischer Mittel, beispielsweise Cremes, Lotionen oder Salben, werden öllösliche Grundlagen benötigt, deren Aufgabe es ist, polare Inhaltssstoffe, wie beispielsweise Wirkstoffe oder Feuchtigkeit, durch die Lipidbarriere der Haut zu transportieren. Für diesen Zweck eignen sich eine Vielzahl von natürlichen und synthetischen ölen, beispielsweise Mandel- oder Avocadoöl oder Esteröle auf Basis von kurzkettigen Triglyceriden. Den ölkomponenten in den angesprochenen Mitteln kommt gleichzeitig auch eine pflegende Wirkung zu, die in unmittelbarem Zusammenhang mit der Hautfettung steht. Vom Konsumenten gewünscht sind Produkte, die ein nichtklebriges, möglichst rasch eintretendes und längeranhaltendes Gefühl der Hautglätte- und -geschmeidigkeit vermitteln.

Das subjektive Empfinden auf der Haut kann mit dem physikochemischen Parameter der Spreitung der Ölkörper auf der Haut korreliert und objektiviert werden, wie dies von U.Zeidler in **Fette, Seifen, Anstrichmitt. 87, 403 (1985)** dargestellt worden ist. Demnach lassen sich kosmetische Ölkörper in niedrigspreitende (unter 300 mm²/10min), mittelspreitende (ca. 300 bis 1000 mm²/10min) und hochspreitende Öle (oberhalb 1000 mm²/10min) einteilen.

Setzt man in einer vorgegebenen Formulierung als Ölkörper ein hochspreitendes Öl ein, so wird zwar sehr rasch das gewünschte Gefühl der Hautglättung erzielt, das Erlebnis dauert jedoch nicht lange an. Umgekehrt wird bei Verwendung niedrigspreitender Öle ein nur wenig ausgeprägtes Glättegefühl erzielt, welches über einen längeren Zeitraum praktisch unverändert erhalten bleibt und daher ebenfalls unbefriedigend ist. Die naheliegende Kombination eines niedrig- und eines hochspreitenden Öles führt erstaunlicherweise nicht zur angestrebten additiven Wirkung, man nimmt vielmehr subjektiv zu Beginn die starke Glättewirkung der schnellspreitenden Ölkomponente und dann ganz unabhängig den Effekt des langsam spreitenden Öles wahr. In der Praxis müssen daher üblicherweise komplexe, genau aufeinander abgestimmte Mischungen von Ölkörpern eingesetzt werden, die im Sinne einer Kaskade ihre Wirkung nacheinander entfalten ohne sich dabei gegenseitig negativ zu beeinflussen. Es liegt auf der Hand, daß die Entwicklung derartiger Systeme mit hohem technischen Aufwand verbunden und damit auch aus ökonomischer Sicht verbesserungswürdig ist [vgl. A.Ansmann, **SÖFW-Journal, 120, 158 (1994)].**

Die Aufgabe der Erfindung hat somit darin bestanden, neue kosmetische und/oder pharmazeutische Zubereitungen zur Verfügung zu stellen, die frei von den geschilderten Nachteilen sind.

### Beschreibung der Erfindung

Gegenstand der Erfindung sind kosmetische und/oder pharmazeutische Zubereitungen mit verbessertem Hautgefühl, die sich dadurch auszeichnen, daß sie Guerbetalkohole auf Basis von Fettalkoholgemischen mit 6 bis 12 Kohlenstoffatomen und/oder deren Ester mit Fettsäuren mit 6 bis 22 Kohlenstoffatomen enthalten, wobei die Spreitung der Ölkomponenten nach Zeidler in Summe im Bereich von ca. 300 bis 1000 mm²/10min liegt.

Überraschenderweise wurde gefunden, daß kosmetische bzw. pharmazeutische Zubereitungen, die als Ölkörper Guerbetalkohole auf Basis von Fettalkoholmischungen der genannten Kettenlänge und/oder deren Ester enthalten, das gewünschte unmittelbare und längeranhaltende Gefühl einer Hautglättung vermitteln. Setzt man an Stelle dieser Guerbetalkohole hingegen Guerbetalkohole auf Basis der reinen Fettalkohole ein oder mischt die Guerbetalkohole der reinen Fettalkohole entsprechend ab, wird ein signifikant schlechteres Hautgefühl erreicht.

Bei der Guerbetisierung von Alkoholgemischen werden zwangsläufig auch gemischte Guerbetalkohole gebildet. Derartige Mischverbindungen sind weder in solchen Guerbetalkoholen enthalten, die man durch Guerbetisierung der reinen Fettalkohole, noch durch Abmischnung von Guerbetalkoholen auf Basis reiner Fettalkohole enthält. Die Erfindung schließt jedoch die Erkenntnis ein, daß gerade dieser Anteil von Mischverbindungen, der ausschließlich durch Guerbetisierung von Fettalkoholmischungen erhalten wird, für die überraschenden hautkosmetischen Eigenschaften der Ölkörper verantwortlich ist.

Die Erfindung schließt des weiteren die Erkenntnis ein, daß der gewünschte Effekt der Hautglättung noch weiter verbessert werden kann, wenn man die genannten Guerbetalkohole mit weiteren Ölkörpern, vorzugsweise Estern von Fettsäuren mit 2-Ethylhexanol in einer solchen Weise abmischt, daß der Spreitwert der Ölmischung dem eines mittelspreitenden Öls entspricht und nach Zeidler einen Wert im Bereich von 350 bis 900 mm²/10min aufweist. Schon hier sei darauf hingewiesen, daß die Verwendung eines mittelspreitenden Öls oder einer Ölmischung, deren Spreitung der eines mittelspreitenden Öls entspricht, ohne Zusatz der Guerbetalkohole auf Basis von Fettalkoholmischungen ebenfalls nicht ohne weiteres zu dem angestrebten Hautglättegefühl führt.

### Guerbetalkohole

Guerbetalkohole stellen bekannte Ölkörper dar, die üblicherweise durch basenkatalysierte Kondensation von Fettalkoholen hergestellt werden. Eine Übersicht zu diesem Thema ist von A.J.O'Lennick und R.E.Bilbo in **Soap Cosm.Chem.Spec. April, 52 (1987)** erschienen.

Im Sinne der Erfindung kommen als Ölkörper Guerbetalkohole auf Basis von Fettalkoholen mit 6 bis 12, vorzugsweise 8 bis 10 Kohlenstoffatomen in Betracht. Typische Beispiele sind Guerbetalkohole auf Basis von Mischungen enthaltend Capronalkohol, Caprylalkohol, Caprinalkohol und/oder Laurylalkohol. Eine typische Fettalkoholmischung innerhalb des angegebenen C-Zahlbereiches stellt der sogenannte C_{8/10}-Vorlauffettalkohol dar, der als Schnitt bei der Destillation beispielsweise von Kokos-, Palm- oder Palmkernöl hergestellt wird. Besonders bevorzugt ist ein Guerbetalkohol auf Basis eines Fettalkohols mit einer C-Kettenverteilung von < 5 Gew.-% C₆, 50 bis 60 Gew.-% C₈, ca. 35 bis 45 Gew.-% C₁₀ und < 2 Gew.-% C₁₂.

### Guerbetester

Die oben genannten Guerbetalkohole können auch in Form ihrer Ester mit Fettsäuren mit 6 bis 22, vorzugsweise 8 bis 18 und insbesondere 12 bis 14 Kohlenstoffatomen eingesetzt werden. Typische Beispiele sind Ester der Guerbetalkohole mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Die Guerbetester können einen Veresterungsgrad von 100 % aufweisen, vorzugsweise gelangen jedoch Partialester zum Einsatz, die einen Veresterungsgrad im Bereich von 20 bis 90 und insbesondere 40 bis 70 % der Theorie aufweisen. Besonders bevorzugt sind Ester auf Basis der oben genannten Guerbetalkohole mit technischen C₈-C₁₈- bzw. C₁₂-C₁₄-Kokosfettsäuren, die einen Veresterungsgrad im Bereich von 40 bis 70 aufweisen. Ebensogut können entsprechende Mischungen von vollständig veresterten Guerbetestern und den entsprechenden Guerbetalkoholen im Gewichtsverhältnis 10 : 90 bis 90 : 10, vorzugsweise 30 : 70 bis 70 : 30 eingesetzt werden.

### Ölkörper

Die erfindungsgemäßen Zubereitungen können weitere Ölkörper enthalten, deren Auswahl solange unkritisch ist, wie die Mischung der Ölkörper mit den Guerbetalkoholen auf Basis von kurzkettigen Fettalkoholgemischen eine Spreitung nach Zeidler im Bereich eines mittelspreitenden Öles, d.h. von ca. 300 bis 1000 mm²/10 min ergibt. Beispiele für in den Zubereitungen über die genannten Guerbetalkohole hinaus einsetzbare Ölkörper sind:
b1) Ester von linearen C₆-C₂₀-Fettsäuren mit linearen C₆-C₂₀-Fettalkoholen,
b2) Ester von verzweigten C₆-C₁₃-Carbonsäuren mit linearen C₁₆-C₁₈-Fettalkoholen,
b3) Ester von linearen C₁₀-C₁₈-Fettsäuren mit verzweigten Alkoholen, insbesondere 2-Ethylhexanol,
b4) Ester von linearen und/oder verzweigten Fettsäuren mit zweiwertigen Alkoholen,
b5) Triglyceride auf Basis C₆-C₁₀-Fettsäuren,
b6) pflanzliche Öle,
b7) verzweigte primäre Alkohole,
b8) substituierte Cyclohexane und/oder
b9) Dialkylether.

Typische Beispiele hierfür sind Ester von Caprin- und Caprylsäure mit Kokosfettalkohol, Ester von Kokos- oder Palmkernfettsäure mit 2-Ethylhexanol, Ölsäuredecylester, Isononansäurecetearylester, Triglyceride auf Basis von Caprin/Caprylsäure, Isocetylalkohol, Di-n-octylcyclohexan, Di-i-octylcyclohexan, Di-n-octylether und Di-2-ethylhexylether.

Als ebenfalls sehr vorteilhaft hat sich die Kombination aus den genannten Guerbetalkoholen und Fettsäure-2-ethylhexylestern erwiesen. Aus sensorischer Sicht ist der Einsatz einer Mischung aus einem Guerbetalkohol auf Basis einer Mischung von Fettalkoholen mit 6 bis 12 Kohlenstoffatomen und einem 2-Ethylhexylester auf Basis einer Fettsäure mit folgender C-Kettenverteilung besonders vorteilhaft: < 3 Gew.-% C₁₄, 45 bis 53 Gew.-% C₁₆, 43 bis 52 Gew.-% C₁₈ und < 2 Gew.-% C₁₈.

### Zusammensetzung der Mittel

Die erfindungsgemäßen kosmetischen und/oder pharmazeutischen Mittel können a) die Guerbetalkohole und/oder Guerbetester und b) weitere Ölkörper mit der einschränkenden Maßgabe des Spreitbereiches im Gewichtsverhältnis 100 : 0 bis 10 : 90, vorzugsweise 90 : 10 bis 10 : 90 und insbesondere 70 : 30 bis 30 : 70 enthalten. Die Herstellung der Mittel kann in an sich bekannter Weise, d.h. beispielsweise durch Heiß-, Kalt-, Heiß-Heiß/Kalt- bzw. PIT- Emulgierung erfolgen. Hierbei handelt es sich um ein rein mechanisches Verfahren, eine chemische Reaktion findet nicht statt.

Beispiele für Mischungen der genannten Art sind Abmischungen eines Guerbetalkohols auf Basis einer C₆-C₁₂-Fettalkohol-Mischung mit C₁₆/₁₈-Fettsäure-2-ethylhexylester, Ölsäuredecylester, Ölsäureoleylester, Triglyceriden auf Basis C_{8/10}-Fettsäure oder Isononansäurecetearylester im Gewichtsverhältnis 50 : 50. Weitere Beispiele sind Abmischungen eines Guerbetalkohols auf Basis einer C₆-C₁₂-Fettalkohol-Mischung mit Mandelöl im Gewichtsverhältnis 90 : 10 bzw. Di-n-octylether im Gewichtsverhältnis 80 : 20.

Ferner können die Mittel, wie beispielsweise Cremes und Lotionen, einen Gehalt an Emulgatoren, Fetten und Wachsen, Stabilisatoren sowie Überfettungsmitteln, Verdickungsmitteln, biogenen Wirkstoffen, Filmbildnern, Konservierungsmitteln, Farb- und Duftstoffen aufweisen.

Als **Emulgatoren** kommen sowohl bekannte W/O- als auch O/W-Emulgatoren wie beispielsweise gehärtetes und ethoxyliertes Ricinusöl, Polyglycerinfettsäureester oder Polyglycerinpolyricinoleate bzw. Polyglycerinpoly-12-hydroxystearate in Frage.

Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage.

Als **Stabilisatoren** können Metallsalze von Fettsäuren wie z.B. Magnesium-, Aluminium und/oder Zinkstearat eingesetzt werden.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate und ähnliche Verbindungen.

Als **Konservierungsmittel** eignen sich beispielsweise Phenoxyethanol, Formaldehydlösung, Parabene, Pentandiol oder Sorbinsäure.

Als **Perlglanzmittel** kommen beispielsweise Glycoldistearinsäureester wie Ethylenglycoldistearat, aber auch Fettsäuremonoglycolester bzw. Fettsäuren in Betracht.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen.

### Gewerbliche Anwendbarkeit

Die erfindungsgemäßen kosmetischen und/oder pharmazeutischen Zubereitungen können O/W- oder W/O-Emulsionen, multiple W/O/W-Emulsionen sowie rein lipophile Öle oder Gele darstellen. Sie verfügen über fettende Eigenschaften und vermitteln ein rasches, langanhaltendes und gleichmäßig abnehmendes Hautglättegefühl. Das Problem der "Glättungslücke" wird zuverlässig vermieden. Die Guerbetalkohole auf Basis kurzkettiger Fettalkoholgemische und/oder die entsprechenden Ester können in den Zubereitungen in Mengen von 1 bis 99, in den Emulsionen vorzugsweise in Mengen von 5 bis 30 Gew.-%-bezogen auf die Zubereitungen - enthalten sein.

Die folgenden Beispiele sollen den Gegenstand der Erfindung näher erläutern.

### Beispiele

### I. Spreitwerte nach Zeidler

Die Spreitwerte verschiedener ölkörper wurden nach der Zeidler-Methode durchgeführt. Hierzu wurden jeweils 4 mg der Ölkomponente auf die vorher mit ethanolfeuchter Watte abgeriebene Unterarminnenseite eines Probanden aufgetragen. Die Klimawerte betrugen 23°C und 60 % relative Feuchtigkeit. Nach 10 min wurde das gespreitete Lipid mit Transparentpapier abgedrückt und den Flächeninhalt bestimmt (vgl.Tabelle 1).

**Tabelle 1**

| Spreitung (S) von Ölkörpern [mm²/10min] | | |
|---|---|---|
| **Bsp.** | **Ölkörper** | **S** |
| B1 | Mandelöl | 200 |
| B2 | C_{16/18}-Fettsäure-2-ethylhexylester | 800 |
| B3 | Guerbetalkohol auf Basis Caprylalkohol [Eutanol^{(R)} G 16] | 700 |
| B4 | Guerbetalkohol auf Basis Caprinalkohol | 600 |
| B5 | Ölsäuredecylester [Cetiol^{(R)} V] | 700 |
| A1 | Guerbetalkohol, Basis C_{6/10}-Fettalkohol | 650 |
| A2 | Guerbetalkohol, Basis C_{6/10}-Fettalkohol-C_{12/14}-Kokosfettsäureester | 730 |
| B6 | Triglycerid auf Basis C_{8/10}-Fettsäure [Myritol^{(R)} 318] | 550 |
| B7 | Isononansäure-cetearylester [Cetiol^{(R)} SN] | 700 |
| B8 | Di-n-octylether [Cetiol^{(R)} OE] | 1600 |

### II. Subjektive Beurteilung durch ein Testpanel

Ein Panel bestehend aus 5 erfahrenen Personen testete Formulierungen auf Basis einer Standardhautcreme, die verschiedene Ölkörpermischungen enthielten, auf ihr subjektives Hautglättegefühl. Zugrunde gelegt wurde eine Skala zwischen 1 (kaum Glättung bzw. rasche Abnahme des Gättegefühls) und 6 (schnelles, gleichmäßiges Glättegefühl).

Die Angaben in Tabelle 2 stellen Mittelwerte dar; die Beispiele 1 bis 11 sind erfindungsgemäß, die Beispiele V1 bis V9 dienen dem Vergleich.

**Tabelle 2**

| Subjektives Hautglättegefühl | | | | |
|---|---|---|---|---|
| **Bsp.** | **Komponente 1** | **Komponente 2** | **Verh.** | **Hautglätte** |
| 1 | A1 | - | 100: 0 | 5,1 |
| 2 | A1 | B1 | 90: 10 | 5,2 |
| 3 | A1 | B2 | 50: 50 | 5,8 |
| 4 | A1 | B5 | 50: 50 | 5,7 |
| 5 | A1 | B6 | 50: 50 | 5,2 |
| 6 | A1 | B7 | 50: 50 | 5,2 |
| 7 | A1 | B8 | 80: 20 | 5,4 |
| 8 | A2 | - | 100: 0 | 5,1 |
| 9 | A1 | A2 | 50: 50 | 5,8 |
| 10 | A1 | A2 | 70: 30 | 5,6 |
| 11 | A1 | A2 | 30: 70 | 5,4 |

**Tabelle 2**

| Subjektives Hautglättegefühl (Forts.) | | | | |
|---|---|---|---|---|
| **Bsp.** | **Komponente 1** | **Komponente 2** | **Verh.** | **Hautglätte** |
| V1 | - | B1 | 0:100 | 1,5 |
| V2 | - | B2 | 0:100 | 3,5 |
| V3 | - | B3 | 0:100 | 3,0 |
| V4 | - | B4 | 0:100 | 3,0 |
| V5 | B3 | B4 | 50: 50 | 3,0 |
| V6 | - | B5 | 0:100 | 3,0 |
| V7 | - | B6 | 0:100 | 3,0 |
| V8 | - | B7 | 0:100 | 3,0 |
| V9 | - | B8 | 0:100 | 2,0 |

Oberhalb eines Mittelwertes von 5,0 liegt ein ausgezeichnetes Hautglättungsgefühl vor, unterhalb von 5,0 ist das Ergebnis unbefriedigend. Die Ergebnisse zeigen, daß ein zufriedenstellendes Ergebnis nur bei Einsatz von Guerbetalkoholen auf Basis von kurzkettigen Fettalkoholgemischen bzw. deren Ester sowie Abmischungen dieser Guerbetalkohole mit weiteren Ölkomponenten innerhalb des beanspruchten Spreitbereiches erzielt wird.

## Patentansprüche

1. Kosmetische und/oder pharmazeutische Zubereitungen mit verbessertem Hautgefühl, **dadurch gekennzeichnet,** daß sie Guerbetalkohole auf Basis von Fettalkoholgemischen mit 6 bis 12 Kohlenstoffatomen und/oder deren Ester mit Fettsäuren mit 6 bis 22 Kohlenstoffatomen enthalten, wobei die Spreitung der Ölkomponenten nach Zeidler in Summe im Bereich von ca. 300 bis 1000 mm²/10min liegt.

2. Zubereitungen nach Anspruch 1, **dadurch gekennzeichnet,** daß sie Partialester von Guerbetalkoholen auf Basis von Fettalkoholgemischen mit 6 bis 12 Kohlenstoffatomen mit Fettsäuren mit 12 bis 14 Kohlenstoffatomen enthalten, wobei der Veresterungsgrad im Bereich von 20 bis 90 % liegt.

3. Zubereitungen nach den Ansprüchen 1 und 2, **dadurch gekennzeichnet,** daß sie als weitere ölkörper Fettsäureester des 2-Ethylhexanols enthalten.

## Claims

1. Cosmetic and/or pharmaceutical formulations with an improved feeling on the skin, characterized in that they contain Guerbet alcohols based on fatty alcohol mixtures containing 6 to 12 carbon atoms and/or esters thereof with fatty acids containing 6 to 22 carbon atoms, the total spreading of the oil components as determined by the Zeidler method being in the range from about 300 to 1000 mm²/10 mins.

2. Formulations as claimed in claim 1, characterized in that they contain partial esters of Guerbet alcohols based on fatty alcohol mixtures containing 6 to 12 carbon atoms with fatty acids containing 12 to 14 carbon atoms, the degree of esterification being in the range from 20 to 90%.

3. Formulations as claimed in claims 1 and 2, characterized in that they contain fatty acid esters of 2-ethyl hexanol as additional oils.

## Revendications

1. Préparations cosmétiques et/ou pharmaceutiques donnant un sentiment amélioré sur la peau,
caractérisées en ce qu'
elles contiennent des alcools de Guerbet à base de mélanges d'alcools gras comportant de 1 à 6 atomes de carbone et/ou de leurs esters avec des acides gras comportant de 6 à 22 atomes de carbone, la diffusion des composants huileux selon Zeidler se situant au total dans un intervalle d'environ 300 à 1000 mm²/10 minutes.

2. Préparations selon la revendication 1,
caractérisées en ce qu'
elles contiennent des esters partiels d'alcools de Guerbet à base de mélanges d'alcools gras comportant de 6 à 12 atomes de carbone avec des acides gras comportant de 12 à 14 atomes de carbone, le degré d'estérification se situant dans un intervalle de 20 à 90 %.

3. Préparations selon les revendications 1 et 2,
caractérisées en ce qu'
elles contiennent comme autres corps huileux des esters d'acides gras du 2-éthyl-hexanol.
